(19) Europäisches Patentamt
European Patent Office
Office européen des brevets

(11) Veröffentlichungsnummer: **0 173 026**
**B1**

(12) # EUROPÄISCHE PATENTSCHRIFT

(45) Veröffentlichungstag der Patentschrift: **12.10.88**

(51) Int. Cl.⁴: **C 07 C 49/175**, C 07 C 45/58, C 07 C 45/51, C 07 D 319/12

(21) Anmeldenummer: **85108092.9**

(22) Anmeldetag: **29.06.85**

(54) **Verfahren zur Herstellung der Alkylether und Enolether des Acetoins.**

(30) Priorität: **30.08.84 DE 3431877**

(43) Veröffentlichungstag der Anmeldung: **05.03.86 Patentblatt 86/10**

(45) Bekanntmachung des Hinweises auf die Patenterteilung: **12.10.88 Patentblatt 88/41**

(84) Benannte Vertragsstaaten: **BE CH DE FR GB LI NL**

(56) Entgegenhaltungen:
**DE-A-566 156**
**US-A-3 433 806**
**US-A-3 480 632**

**RESEARCH DISCLOSURE, Nr. 115, November 1973, Seiten 5-6, Nr. 11504; "Isomerization of epoxy compounds (C07c)"**

(73) Patentinhaber: **HÜLS AKTIENGESELLSCHAFT, - RSP Patente / PB 15 - Postfach 13 20, D-4370 Marl 1 (DE)**

(72) Erfinder: **Brockhaus, Rudolf, Dr., Holsteiner Strasse 19, D-4370 Marl (DE)**
Erfinder: **Franke, Hans- Jürgen, Freiheitstrasse 13, D-4270 Dorsten (DE)**

**EP 0 173 026 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

3-Methoxybutanon-(2) (Formel I), der Methylether des Acetoins, ist ein wertvolles Zwischenprodukt, das für eine Reihe von Synthesen geeignet ist. Durch Überführen des 3-Methoxybutanon-(2) in einen Enolether wird die Möglichkeit für gezielte Synthesen noch erweitert.

So gelangt man vom 3-Methoxybutanon-(2) nach klassischer Cyanhydrinsynthese zu einem Nitril, das weiteren Reaktionen zugänglich ist. Durch Methanolabspaltung vom 3-Methoxybutanon-(2) entsteht Methylvinylketon, das z. B. als ein Einsatzstoff für Vitamin A oder für Fungizide dient.

Die Herstellung von 3-Methoxybutanon-(2) ist bisher nur durch Laborsynthese,z. B. ausgehend von 3-Brombutanon-(2), beschrieben (Aston, Mitarb., Am. Soc 64 (1942) 300; Beilstein E III 1 Seite 3210). Dieser Weg ist jedoch sehr aufwendig.

Hieraus ergab sich die Aufgabe, ein einfaches Verfahren zu finden, das die Herstellung von Alkylethern und Enolethern des Acetoins in hoher Ausbeute ermöglicht.

Diese Aufgabe wird erfindungsgemäß entsprechend den Angaben der Patentansprüche gelöst.

Es wurde überraschenderweise gefunden, daß sich 2-Alkoxy-3,3-dimethyloxiran (Epoxid, Formel II, die Methoxy-Verbindung) oder das daraus durch Alkoholanlagerung hergestellte 2-Hydroxy-2-methyl-propanaldialkylacetal (Acetal, Formel III, das Dimethylacetal) unter Spaltbedingungen, die man z. B. bei Dehydratisierungen anwendet, zu 3-Alkoxybutanon-(2) umlagert, indem man entweder das oben genannte Oxiranderivat oder das daraus abgeleitete Acetal dampfförmig ggf. in Gegenwart von oberflächenaktiven Substanzen auf 150 bis 320° C, vorzugsweise 200 bis 300° C, insbesondere 235 bis 270° C bevorzugt bei einer Verweilzeit von 1 bis 22 Sekunden, insbesondere 2 bis 7 Sekunden, erhitzt. Weiterhin wurde gefunden, daß sich 2,5-Dialkoxy-3,3,6,6-tetramethyldioxan-(1,4) (Formel IV, die Dimethoxyverbindung), Dimerisationsprodukt des 2-Alkoxy-3,3-dimethyloxirans, unter den genannten thermischen oder thermisch-katalytischen Bedingungen in 2-Alkoxy-3,3,5,6-tetramethyl-1,4-diox-5-en (Formel V, die Methoxyverbindung) umlagert.

Die erfindungsgemäße Isomerisierung erfolgt in zweifacher Weise:

a) Die Epoxidumlagerung zum Keton.

b) Die Skelettumlagerung des iso-$C_4$-Gerüstes in ein n-$C_4$-Gerüst.

Diese gleichzeitige Umlagerung von a) und b) ist neu. Aus der DE-A-566 156 war es zwar bekannt, ein Epoxid nach (a) zum entsprechenden Keton umzulagern, die Skelettumlagerung nach (b) tritt jedoch nicht ein, obwohl der Epoxidring ebenfalls an einem tert. C-Atom befindet.

Mit diesem erfindungsgemäßen Verfahren gelingt es, technisch leicht zugängige Produkte in wertvolle Folgeprodukte umzuwandeln.

2-Methoxy-3,3-dimethyloxiran ist ein Produkt, das sich glatt durch Luft- oder Sauerstoffoxydation des aus Isobutyraldehyd und Methanol hergestellten Enolethers herstellen läßt. Isobutyraldehyd (IBA) ist eine technisch reichlich zur Verfügung stehende Chemikalie.

Das Oxiran neigt unstabilisiert (stabilisierend wirkt Lauge, z. B. NaOH) zur Dimerisation und bildet ein Dioxanderivat. Für manche Zwecke, auch aus Gründen der Sicherheit (Epoxide neigen zu Spontanreaktion), ist es sinnvoll, aus dem Epoxid zunächst das Acetal herzustellen.

Beide genannten Derivate reagieren unter Skelettisomerisierung zum Keton bzw. dessen Enolether.

Der Reaktionsaufwand für die Umlagerung ist gering, es genügt verdampfen, erhitzen, vorteilhafterweise in Gegenwart von oberflächenaktiven Substanzen, wie sie z. B. zur Wasserabspaltung aus Alkoholen benutzt werden. Besonders gute Ausbeuten werden an $SiO_2$- und $Al_2O_3$-Kontakten mit Laugedotierung erzielt. Als Laugen sind beispielsweise NaOH oder KOH geeignet. Man setzt sie in Mengen von 0,1 bis 2 %, bezogen auf den Kontakt, zu. Säuredotierung des Trägers bewirkt eher eine Verschlechterung der Ausbeute.

Man kann zum besseren Verdampfen und Transport ein Inertgas wie $N_2$ zusetzen. Die Menge Inertgas richtet sich nach dem Dampfdruck. Beim 2-Methoxy-3,3-dimethyloxiran werden beispielsweise 0 bis 60 Vol.-% $N_2$ zugesetzt. Beim schwerer flüchtigen Dioxanderivat werden bis 80 Vol.-% $N_2$ zugesetzt. Anstelle der Methylethergruppe in der Oxiranverbindung oder im Acetal kann auch eine homologe Alkylgruppe, wie zum Beispiel eine Ethyl- oder Propylgruppe, Molekülbestandteil sein. Die Methylverbindung ist am billigsten und bildet weniger Nebenprodukte als Ethyl- oder Propylgruppen. Bevorzugt setzt man daher 2-Methoxy-3,3-dimethyloxiran, 2-Hydroxy-2-methyl-propanaldimethylacetal oder das 2,5-Dimethoxy-3,3,6,6-tetramethyldioxan-(1,4) ein.

Bei der Umlagerung des Dioxanderivates wird Alkanol, z. B. Methanol abgespalten und es entsteht eine Doppelbindung. Die Umlagerung findet überraschenderweise nur auf einer Halbseite des Dioxanmoleküls statt.

Nachfolgend die Formeln der aufgeführten Verbindungen:

I 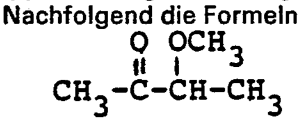 3-Methoxybutanon-(2)

II 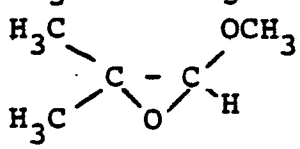 2-Methoxy-3,3-dimethyloxiran (Epoxid)

III 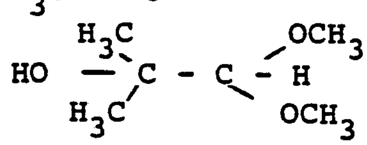 2-Hydroxy-2-methyl-propanaldimethylacetal (Acetal)

IV 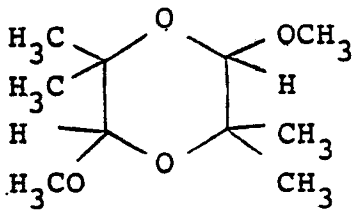 2,4-Dimethoxy-3,3,6,6-tetramethyl-dioxan-(1,4)

V 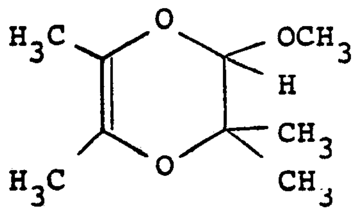 2-Methoxy-3,3,5,6-tetramethyl-1,4-diox-5-en (Enolether des Acetoins)

Die angegebenen Reaktionsbedingungen sind variabel. Es ist allgemeines Grundwissen, daß sich z. B. die Verweilzeit verringern läßt, wenn zum Ausgleich die Reaktionstemperatur erhöht wird. Ebenso beeinflussen apparative Gegebenheiten, Rohrdurchmesser etc. den Reaktionsablauf. Auch unter thermischen Bedingungen z. B. in Gegenwart von Glaskugeln findet die Umlagerung statt. Oberflächenaktive Körper beschleunigen die Reaktion und erlauben schonendere Reaktionsbedingungen, d. h. die Selektivität der Reaktion steigt.

Beschreibung der Versuche:

**Beispiel 1**

Herstellung von 3-Methoxybutanon-(2) durch Umlagerung von 2-Methoxy-3,3-dimethyloxiran (Epoxid)

In einem salzbadbeheizten VA-Rohr-Spiralreaktor von 5 m Länge und 10 mm Durchmesser füllt man 250 ml eines mit 0,1 Gew.-% NaOH-imprägnierten $SiO_2$-Katalysators; Stranggröße: Durchmesser = 4 mm, Länge = 6 mm.[1])

Bei einer Salzbadtemperatur von 235° C gibt man stündlich 500 g Epoxid roh (ca. 96 bis 98 %-ig) gasförmig über den Katalysator. Die Verweilzeit bezogen auf das leere Rohr, beträgt 3 sec. Gleichzeitig gibt man einen Inertgasstrom ($N_2$) von ca. 50 l/h über den Katalysator. Der kondensierbare Anteil des Reaktionsaustrages (498 g) setzt sich wie folgt zusammen:

0,1 % Methanol
0,4 % Aceton
1,4 % Methylvinylketon
3,66 % Epoxid
93,4 % 3-Methoxybutanon-(2)
0,9 % Sonstige

Daraus errechnet sich ein Epoxid-Umsatz von 96,3 % und eine 3-Methoxybutanon-(2)-Ausbeute von 98,5 %.

Der Reaktionsaustrag wurde über eine 30 cm-Kolonne, gefüllt mit 4 x 4 mm Drahtnetzringen, bei 180 mbar destilliert. Bei einem Rücklaufverhältnis von 1 : 10 und einer Temperatur in der Dampfphase von 67° C (180 mbar) konnten ≦ 95 % des 3-Methoxybutanon-(2) mit einer Reinheit von = 99 % isoliert werden.

**Beispiele 2 bis 15**

Apparatur und Durchführung wie in Beispiel 1.

In den Beispielen 2 bis 11 wurden als Katalysatorenl [1]) $Al_2O_3$-Stränge, Durchmesser = 4 mm, Länge = 4 mm mit und ohne KOH-Dotierung, $SiO_2$-Stränge, Durchmesser = 4 mm, Länge = 6 mm mit und ohne NaOH Dotierung, im Beispiel 12 3 mm Glasperlen, im Beispiel 13 V4A-Raschigringe, Durchmesser = 4 mm, Länge = 4 mm und im Beispiel 15 die o. g. $SiO_2$-Stränge mit $P_2O_5$-Dotierung, eingesetzt. Beispiel 14 wurde ohne Reaktorfüllung (Leerrohrversuch) gefahren. Die Beispiele 5 und 12 wurden ohne Inertgaszusatz gefahren. Dabei wurden die Reaktionstemperatur sowie die Durchsatzmenge variiert.

Die Ergebnisse der einzelnen Beispiele sind in der Tabelle 1 zusammengefaßt.

**Beispiel 16**

Herstellung von 3-Ethoxybutanon-(2) durch Umlagerung von 2-Ethoxy-3,3-dimethyloxiran (Epoxid)
Apparatur, Katalysator und Durchführung wie in Beispiel 1.

Bei einer Salzbadtemperatur von 235° C gibt man stündlich 300 g 97,2 %-iges Epoxid gasförmig über den Katalysator. Gleichzeitig gibt man einen Inertgasstrom ($N_2$) von ca. 50 l/h über den Katalysator. Der Reaktionsaustrag wird kondensiert. Im Kondensat sind 11,9 g nicht umgesetztes Epoxid und 271,6 g 3-Ethoxybutanon-(2), was einem Epoxid-Umsatz von 95,9 % und einer 3-Ethoxybutanon-(2)-Ausbeute von 97,1 % entspricht.

**Beispiel 17**

Herstellung von 3-Propoxybutanon-(2) durch Umlagerung von 2-Propoxy-3,3-dimethyloxiran (Epoxid)
Apparatur, Katalysator und Durchführung wie in Beispiel 1.

300 g Epoxid, 96,3 %-ig und 50 l $N_2$ gibt man stündlich gasförmig bei 235° C über den Katalysator. Der Reaktionsaustrag enthält 11,3 g nicht umgesetztes Epoxid und 260,4 g 3-Propoxybutanon-(2), woraus sich ein Epoxid-Umsatz von 96,1 % und eine 3-Propoxybutanon-(2)-Ausbeute von 93,8 % errechnet.
Die Ergebnisse der Beispiele 16 und 17 sind in Tabelle 2 zusammengefaßt.

**Beispiele 18 und 19**

Herstellung von 3-Methoxybutanon-(2) durch Umlagerung von 2-Hydroxy-2-methylpropanal-dimethylacetal (Acetal) unter Methanolabspaltung
Apparatur und Durchführung wie in Beispiel 1.

In den Beispielen 18 und 19 wurden mit NaOH dotierte $Al_2O_3$-Stränge als Katalysator eingesetzt. Die Reaktionstemperatur war im Beispiel 18 250° C und im Beispiel 19 270° C, der Acetaleinsatz im Beispiel 18 100 g/h und im Beispiel 19 290 g/h (Acetalgehalt = 97 %). Die Ergebnisse der Beispiele 18 und 19 sind in der Tabelle 2 zusammengefaßt.

**Beispiele 20 und 21**

Herstellung von 2-Methoxy-3,3,5,6-tetramethyl-1,4,-diox-5-en aus 2,5-Dimethoxy-3,3,6,6-tetramethyldioxan-(1,4)
Apparatur und Durchführung wie in Beispiel 1.

In den Beispielen 20 und 21 wurden $SiO_2$-Stränge als Katalysator eingesetzt. Bei gleichen Durchsatzmengen (106 g/h 90,5 %-iges 2,5-Dimethoxy-3,3,6,6-tetramethyldioxan-(1,4), Rest Epoxidmonomer) wurden im Beispiel 20 260° C und im Beispiel 21 320° C als Reaktionstemperatur gehalten.
Die Ergebnisse der Beispiele 20 und 21 sind in der Tabelle 2 zusammengefaßt.

[1]) Bemerkungen zu den eingesetzten Katalysatoren:

| | |
|---|---|
| $Al_2O_3$ | : Schüttdichte = 0,717;<br>Porenvolumen = 0,46 $cm^3/g$;<br>Oberfläche = 200 $m^2/g$ |
| $SiO_2$ | : Schüttdichte =0,43;<br>Porenvolumen = 1 $cm^3/g$;<br>Oberfläche = 140 bis 150 $m^2/g$ |
| Glaskugeln | : normales Industrieglas |

Tabelle 1

| Beispiel lfd. Nr. | Reaktions-temp. (°C) | Verweil-zeit (sec.) | Katalysator (250 ml) | Einsatz (g/h) | Umsatz (%) | Ausbeute bzw. auf Umsatz (%) | RZA (g/l Kat. x h) |
|---|---|---|---|---|---|---|---|
| Epoxid zu 3-Methoxybutanon-(2) | | | | | | 3-Methoxybutanon-(2) | |
| 1 | 235 | 3,0 | $SiO_2$ + 0,1 Gew.-% NaOH | 500 | 96,3 | 98,5 | 1 860 |
| 2 | 235 | 1,8 | $SiO_2$ | 966 | 97,6 | 94,1 | 3 480 |
| 3 | 150 | 6,7 | $Al_2O_3$ | 167 | 97,3 | 81,3 | 517 |
| 4 | 205 | 6,0 | $Al_2O_3$ | 163 | 97,7 | 86,1 | 539 |
| 5 | 235 | 13,8 | $Al_2O_3$ | 160 | 97,7 | 86,7 | 531 |
| 6 | 304 | 4,8 | $Al_2O_3$ | 175 | 98,1 | 57,9 | 389 |
| 7 | 298 | 4,9 | $Al_2O_3$ + 0,1 Gew.-% KOH | 174 | 98,0 | 86,0 | 574 |
| 8 | 235 | 5,7 | $Al_2O_3$ + 0,1 Gew.-% KOH | 162 | 96,9 | 93,9 | 577 |
| 9 | 235 | 2,9 | $SiO_2$ + 0,3 Gew.-% NaOH | 532 | 96,1 | 94,2 | 1 849 |
| 10 | 235 | 4,4 | $SiO_2$ + 0,5 Gew.-% NaOH | 278 | 97,0 | 77,2 | 799 |
| 11 | 235 | 2,9 | $SiO_2$ + 2,0 Gew.-% NaOH | 532 | 31,2 | 71,1 | 453 |
| 12 | 235 | 21,2 | Glasperlen | 104 | 90,2 | 87,6 | 322 |
| 13 | 235 | 2,9 | $V_4$A-Raschigringe | 532 | 15,9 | 55,9 | 180 |
| 14 | 300 | 2,6 | -, leeres Rohr | 532 | 22,5 | 80,8 | 371 |
| 15 | 235 | 4,4 | $SiO_2$ + 0,5 Gew.-% $P_2O_5$ | 277 | 97,5 | 79,9 | 828 |

Tabelle 2

| Beispiel lfd. Nr. | Reaktions-temp. (°C) | Verweil-zeit (sec.) | Katalysator (250 ml) | Einsatz (g/h) | Umsatz (%) | Ausbeute bzw. auf Umsatz (%) | RZA (g/l Kat. x h) |
|---|---|---|---|---|---|---|---|
| Epoxid zu 3-Methoxybutanon-(2). | | | | | | 3-Methoxybutanon-(2) | |
| 16 | 235 | 4,5 | $SiO_2$ + 0,1 Gew.-% NaOH | 300 | 95,9 | 97,1 | 1 086 |
| Epoxid zu 3-Propoxtbutanon-(2) | | | | | | 3-Propoxybutanon-(2) | |
| 17 | 235 | 4,8 | $SiO_2$ + 0,1 Gew.-% NaOH | 300 | 96,1 | 93,8 | 1 042 |
| Acetal zu 3-Methoxybutanon-(2) | | | | | | 3-Metoxybutanon-(2) | |
| 18 | 250 | 7,0 | $Al_2O_3$ + 0,1 Gew.-% NaOH | 100 | = 99,0 | 94,6 | 276 |
| 19 | 270 | 4,6 | $Al_2O_3$ + 0,1 Gew.-% NaOH | 290 | = 99,0 | 91,3 | 774 |
| 2,5-Dimethoxy-3,3,6,6-tetramethyldioxan-(1,4) zu 2-Methoxy-3,3,5,6-tetramethyl-1,4-diox-5-en | | | | | | 2-Metaoxy-3,3,5,6-tetramethyl-1,4-diox-5-en | |
| 20 | 260 | 7,5 | $SiO_2$ | 106 | 50,3 | 81,0 | 132 |
| 21 | 320 | 6,7 | $SiO_2$ | 106 | 93,6 | 77,5 | 235 |

**Patentansprüche**

1. Verfahren zur Herstellung der Alkylether und Enolether des Acetoins, dadurch gekennzeichnet, daß man das Epoxid, 2-Alkoxy-3,3-dimethyloxiran, das daraus durch Alkoholanlagerung hergestellte Acetal, 2-Hydroxy-2-methyl-propanal-dialkylacetal, oder das Dimerisierungsprodukt des 2-Alkoxy-3,3-dimethyloxirane, das 2,5-Dialkoxy-3,3,6,6-tetramethyldioxan-(1,4) dampfförmig durch Erhitzen auf 150 bis 320° C in zweifacher Weise isomerisiert, wobei der Epoxidring zum Keton und das iso-$C_4$-Gerüst zum n-C-4-Gerüst umgelagert wird.

2. Verfahren nach Anspruch 1, dadurch gekennzeichnet, daß man die Isomerisierung durch Erhitzen auf 200 bis 300° C, vorzugsweise auf 235 bis 270° C durchführt.

3. Verfahren nach den Ansprüchen 1 und 2, dadurch gekennzeichnet, daß man die Isomerisierung bei einer Verweilzeit von 1 bis 22 Sekunden, vorzugsweise 2 bis 7 Sekunden durchführt.

4. Verfahren nach den Ansprüchen 1 bis 3, dadurch gekennzeichnet, daß man in Gegenwart von oberflächenaktiven Substanzen isomerisiert.

5. Verfahren nach den Ansprüchen 1 bis 4, dadurch gekennzeichnet, daß man als oberflächenaktive Substanzen $SiO_2$- oder $Al_2O_3$-Kontakte, bevorzugt mit Laugedotierung,

einsetzt.

6. Verfahren nach den Ansprüchen 1 bis 5,
dadurch gekennzeichnet,
daß man bei der Isomerisierung ein Inertgas, bevorzugt $N_2$, zusetzt.

## Claims

1. A process for the preparation of the alkyl ether and enol ether of acetoin, characterized in that the epoxide, 2-alkoxy-3,3-dimethyloxirane, the acetal, 2-hydroxy-2-methyl-propanal dialkyl acetal, prepared therefrom by an addition reaction with alcohol, or the dimerization product of 2-alkoxy-3,3-dimethyloxirane, 2,5-dialkoxy-3,3,6,6-tetramethyl-1,4-dioxane, is isomerized in two ways in the vapour phase by heating to a temperature between 150 and 320° C, the epoxide ring rearranging to form the ketone and the iso-$C_4$-structure rearranging to form the n-$C_4$-structure.

2. A process according to claim 1, characterized in that the isomerization is carried out by heating to a temperature between 200 and 300° C, preferably between 235 and 270° C.

3. A process according to either of claims 1 and 2, characterized in that the isomerization is carried out at a residence time of 1 to 22 seconds, preferably 2 to 7 seconds.

4. A process according to any of claims 1 to 3, characterized in that the isomerization is carried out in the presence of a surfactant.

5. A process according to any of claims 1 to 4, characterized in that the surfactant employed is an $SiO_2$ or $Al_2O_3$ catalyst, preferably doped with a lye.

6. A process according to any of claims 1 to 5, characterized in that an inert gas, preferably $N_2$, is added during the isomerization.

## Revendications

1. Procédé de préparation des éthers alkyliques et des éthers énoliques de l'acétoine, caractérisé par le fait que l'on isomérise par deux fois l'époxyde, à savoir, le 2-alkoxy-3,3-diméthyl-oxiranne, l'acétal préparé à partir de ce dernier par addition d'alcool, à savoir le 2-hydroxy-2-méthyl-propanal-dialkylacétal, ou le produit de dimérisation du 2-alkoxy-3,3-diméthyl-oxiranne, sous forme de vapeur, par chauffage a une température de 150 à 320° C, le composé cyclique de résine époxy étant réarrangé en cétone, et le squelette iso-$C_4$ en squelette n-$C_{-4}$.

2. Procédé selon la revendication 1, caractérisé par le fait que l'on effectue l'isomérisation par chauffage à une température de 200 à 300° C, de préference de 235 à 270° C.

3. Procédé selon les revendications 1 et 2, caractérisé par le fait que l'on effectue l'isomérisation avec un temps de séjour de 1 à 22 secondes, de préférence de 2 à 7 secondes.

4. Procédé selon les revendications 1 à 3, caractérisé par le fait que l'on isomérise en présence de substances tensio-actives.

5. Procédé selon les revendications 1 à 4, caractérisé par le fait que l'on utilise, comme substances tensio-actives, des catalyseurs à base de $SiO_2$ ou $Al_2O_3$, de préférence avec dopage à la lessive alcaline.

6. Procédé selon les revendications 1 à 5, caractérisé par le fait que l'on ajoute un gaz inerte, de préférence du $N_2$, lors de l'isomérisation.